# EUROPEAN PATENT APPLICATION

(11) **EP 1 788 498 A1**
(43) Date of publication of application: **23.05.2007**
(21) Application number: 05025351.7
(22) Date of filing: 21.11.2005
(51) Int. Cl.: G06F 19/00, A61M 5/14

(54) **Apparatus for infusing substances**

(71) Applicant: BrainLAB AG, 85551 Kirchheim/Heimstetten (DE)
(72) Inventor: Hartlep, Andreas, Dr., 83629 Weyarn (DE); Pedain, Christoph, Dr., 81667 München (DE); Brady, Martin, Dr., Baltimore, MD 21210 (US); Raghavan, Raghu, Dr., Phoenix, MD 21131 (US)
(74) Representative: Schwabe - Sandmair - Marx

(57) **Abstract**

The present invention relates to method for determining the backflow of a substance or fluid along a track of a delivery device including: a) obtaining parameters that influence the flow of the fluid or substance; b) acquiring information about delivery data; and computing the backflow along the delivery device using the information in steps a) and b).

## Description

The present invention relates to methods and an apparatus for delivering agents, especially for intraparenchymal delivery of therapeutic agents in solution under positive pressure by infusing the agents into a body or tissue.

The present invention relates especially to the planning of the delivery of material into tissue within a subject or patient, whereby the delivery of for example therapeutic, image enhancing, bio-active, pharmacological, nano-technichal or otherwise active materials can be enhanced by calculating or simulating the backflow of the material flowing out of the delivery device or catheter.

Devices to deliver a substance, such as injection or infusion devices, are known in the art and for example described in US 5,720,720 and US 6,572,579 B1.

US 6,549,803 B1 discloses the modelling of material in an organism by a uniformly structured field of static constants.

US 2002/0114780 A1 discloses a method of increasing the volume of distribution of a therapeutic agent in a tissue in a subject during localized delivery.

It is an object of the present invention to provide a method and an apparatus of improving the delivery of a substance, such as drugs, into or through tissue or various tissue structures.

This object is solved by the independent claims. Preferred embodiments are defined in the dependent claims.

According to the present invention the convection-enhanced drug delivery is improved and the predictability and the reliability of drug delivery is increased by accounting for the backflow which is the phenomenon that a fluid or substance infused or injected through a delivery or infusion device, such as a catheter, cannula or needle, flows through one or more openings mostly at the tip or even at the sides of the infusion device and after leaving the delivery device flows along the outside of the delivery device before it begins to flow significantly into the surrounding tissue. The backflow depends on various parameter, such as density or elasticity of the surrounding tissue. The infusion parameters, such as infusion rate, infused material, geometry of the infusion device can be freely chosen and can thus vary from patient to patient. In many cases the diffusion of the substance to be delivered into tissue starts only significantly after the pressure onto the tissue caused by the backflow which is the outflown substance flowing in part back along the delivery device is high enough, so that basically no more backflow along the catehter track occurs and the substance can be diffused into the surrounding tissue. The length of this backflow can be several centimeters for catheters of the diameter of a milimeter under clinical conditions. The present inventors found that the region of backflow, rather than the catheter port(s), acts as the effective source of the infusion, controlling in large part the shape of the subsequent distribution of the substance in tissue.

Thus, since the backflow is strongly effected by the nature of the tissue surrounding the catheter, the tissue is accessed or tissue parameters are acquired according to an embodiment of the invention by for example magnetic resonance imaging, subsequently employing the mathematics of backflow to estimate or even control the backflow length, wherein the process required to plan the delivery can be based on the backflow characteristics, such as backflow length or shape, determined in advance or determined specifically for a patient.

According to the present invention, the determination of the backflow means to determine for example one or more of the following parameters or characteristics, wherein for the purpose of the invention only a single one of the following parameters or two or more of the respective parameters can be determined or used:
- The extension of the backflow, such as the length and/or shape of the backflow in various directions, as for example along the delivery device or catheter path.
- The time dependent distribution or course of the substance exiting the delivery device.
- The behaviour or flow of the delivered substance.
- The geometry or expansion or dilatation of the substance depending on the time after exiting the delivery device.

The method according to the present invention for determining the backflow or parameters of the backflow, such as the length of the backflow along a delivery device, such as a catheter or along a catheter track, includes the steps of: obtaining parameters that influence the fluid flow and acquiring the information about the delivery data, which can be delivery parameters and/or information about the delivery device, such as the geometry or structure of the delivery device, catheter diameter, catheter profile and/or properties of the substance or substances to be delivered, such as fluid properties being e.g.: fluid viscosity or fluid molecular size and/or parameters of the infusion, such as the flow rate or pressure.

The parameters that influence the fluid flow, such as patient-specific data concerning the individual tissue structure, and the delivery data is used to compute the backflow along the delivery device or catheter so that according to the present invention the backflow can be determined or calculated. Depending on the parameters or data used to calculate the backflow, the length or region of the backflow can for example be determined so that for example the backflow region can be used as an effective source of the infusion for further simulation or control of the delivery parameters, such as the number and placement of the catheters on a body. Additionally, the shape of the subsequent distribution of the infused substance in tissue can be simulated or even controlled.

The determination of the backflow in terms of e.g. length or shape is significant for shaping or planing the subsequent infusion and thus, according to the present invention, the infusion shapes and extents can be calculated or simulated based on the actual and probably time dependent shape or geometry of the backflow, wherein the nature of the tissue influencing the backflow length or shapes can be considered by using for example patient-specific medical or anatomical data to determine the type of tissue surrounding the delivery device, such as gray matter, white matter or even the anisotropy of the tissue.

It is preferable that patient specfic medical and/or anatomical data is aquired prior to the computation of the backflow, which patient specific medical data can be: the diffusivity of water moleculs, capillary permeability, blood flow or blood volume.

According to a further aspect of the invention an apparatus for determining the backflow includes an apparatus for acquiring patient-specific medical and/or anatomical and/or physiological data, such as an imaging apparatus for obtaining MRI, CT, PET, SPECT and/or X-Ray images. This apparatus is connected to a data processor or computer to receive the patient specific anatomical or medical data. The data processor is furthermore connected to a data base including information or parameters describing the influence of the patient specific data on the fluid flow, such as the behaviour or characteristics of different types of tissue to specify or simulate for example the density or diffusivity or permeability or blood flow of or through each respective type of tissue. Furthermore, the data base can include generalized information about parameters or properties of different types of tissue which could have been obtained by experience, from literature, by modelling, studies, research or analysis. Additionally, the data base can include parameters of the delivery device and/or properties of the fluid or substance to be delivered. The data processor connected to the imaging apparatus and the data base can then compute the distance or shape of the backflow along the delivery device using the information obtained from the imaging device and preferably also based on the information obtained from the data base. The calculated backflow can then for example be shown on a screen connected to the computer.

The present invention is described with reference to a preferred embodiment, wherein:
- Figure 1: shows a system according to the present invention; and
- Figure 2: shows a flow chart of the present invention.

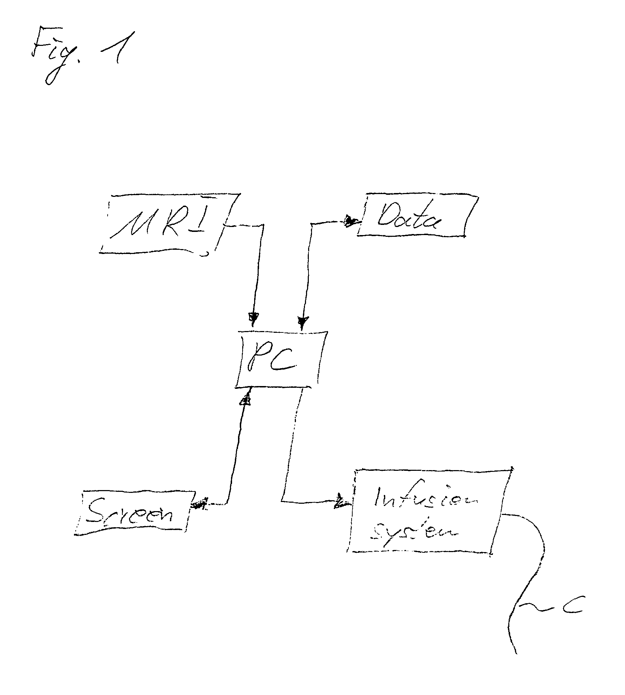

Figure 1 shows an infusion system of the present invention, wherein an MRI apparatus is used as an imaging device which is connected to a computer PC, to obtain patient-specific medical and/or anatomical and/or physiological data. The computer is also connected to a data bank Data to obtain information on the properties of the tissue detected by the imaging device MRI making it for example possible to calculate parameters relevant for drug delivery out of the imaging data delivered by the imaging device MRI, such as the permeability of a specific type of tissue or the metabolism in a specific tissue region or metabolism parameters of a substance or fluid to be delivered. Parameters for planning an infusion, such as the region of backflow, are output to a touch-screen which can be used as an input device for the computer PC to input the selections of a user influencing for example the delivery plan. Furthermore, the computer PC is connected to an infusion system including for example a reservoir for the fluid to be infused and a pump connected to the catheter C, to control for example the placement or insertion of one or more catheters using e.g. the VectorVision system to a desired infusion site or to control delivery parameters, such as the flow rate or pressure of the substance to be delivered, by sending e.g. motor control signal to the pumping device.

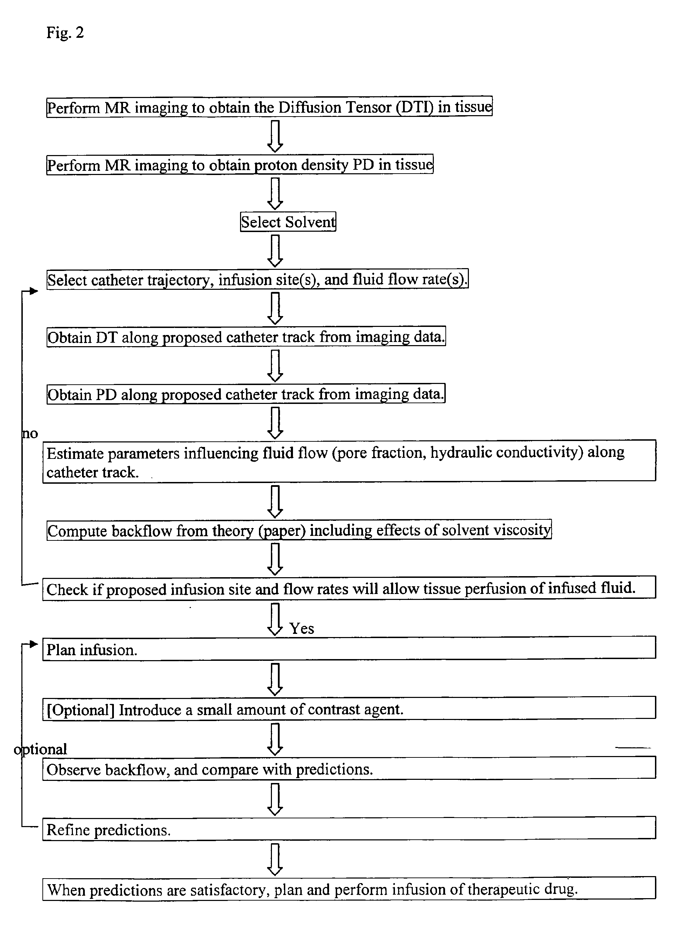

Figure 2 shows a flow chart explaining an embodiment of the inventive method, wherein an MR image is taken to obtain the diffusion tensor DTI in tissue. Furthermore, the proton density PDI in tissue is obtained from an MR image. The solvent to be infused is selected, preferably based on the information of the DTI and/or PD. The catheter tragectory, infusion site(s) and fluid flow rate(s) are selected. From the imaging data the DT and PD are obtained along a proposed catheter track. Parameters influencing the fluid flow, such as pore fraction or hydraulic conductivity are estimated along the proposed catheter track, the backflow is computed from theory including effects of the solvent viscosity to simulate for example the backflow shape to subsequently check if the proposed infusion site and flow rates will allow tissue perfusion of the infused fluid. If this is possible, the infusion is planned, if not, a different catheter tragectory and/or a different infusion site and/or different fluid flow rates are selected and the process to compute the backflow is repeated.

According to a preferred embodiment, a small amount of a contrast agent can be introduced to observe the backflow and to compare the observed backflow with the computed predicted backflow. The result of the observation can be used to refine the predictions and to refine the infusion plan.

When the predictions are satisfactory, the infusion of a therapeutic drug can be planned and performed.

## Claims

1. Method for determining the backflow of a substance or fluid along a track of a delivery device (C) including:
a) obtaining parameters that influence the flow of the fluid or substance;
b) acquiring information about delivery data; and
c) computing the backflow along the delivery device (C) using the information in steps a) and b).

2. The method of claim 1, wherein the parameters that influence the substance of fluid flow are based on the acquisition of patient specific medical and/or anatomical data and/or generalized information derived or drawn from one or more of the following: experience, literature, modeling, studies, research, analysis.

3. The method according to one of the preceeding claims, wherein the information about the delivery data includes one or more of the following: delivery parameters and/or delivery geometry and/or fluid properties, such as: delivery device trajectory, flow rate, pressure, catheter diameter, catheter profile, fluid viscosity, fluid molecular size.

4. A method of any of the previous claims wherein the patient-specific medical data includes one or more of the following: MRI, CT, PET, SPECT, x-ray data.

5. A method of any of the previous claims wherein the patient-specific medical data includes one or more of the following: diffusivity of water molecules, capillary permeability, blood flow, blood volume.

6. A method of any fo the previous claims wherein any of the computations in step c include one or more of the following parameters: pore fraction, intracellular volume fraction, extracellular volume fraction, and/or hydraulic conductivity.

7. A method of any of the previous claims wherein the diffusivity of water molecules within the measurement volume is computed.

8. A method of any of the previous claims wherein one or more of the following values are derived from the patient-specific medical data and/or generalized information: pore fraction, intracellular volume fraction, extracellular volume fraction, hydraulic conductivity, measures for blood brain barrier disruption, degradation rate, washout of fluid.

9. A method of any of the previous claims wherein patient-specific medical data and/or generalized information is used to extract regions and/or structures of special relevance to the procedure, such as surfaces, functional areas, nerve fiber tracks, cavities, intracranial structures that influence fluid flow and/or distribution.

10. A method of claim 9 wherein the extraction of region and/or structures is performed automatically or semi-automatically using models and/or algorithms for surface detection and delineation, atlases, anatomical information, and computerized versions thereof.

11. A method of any of the previous claims wherein the derivation of the reference values refer to literature values and/or properties about the target volume and/or the entire measurement volume of the patient specific medical information.

12. A method of any of the previous claims wherein the derivation of the reference values refer to a model for fluid distribution within tissue.

13. A method of any of the previous claims wherein the derivation of the reference values refer to generalized values derived from a database.

14. A method of infusing a contrast agent such that the distribution of such agent can be detected by means of medical data acquisition, especially MRI, CT, x-ray, ultrasound, SPECT, PET, and observing and/or measuring the backflow length along the catheter track.

15. A method of claim 14 wherein the backflow length is measured by using medical images.

16. A method of claim 14 wherein the backflow length is measured automatically by using an imaging processing algorithms.

17. A method of claim 16 wherein the algorithms are specially adapted to properties of the contrast agents.

18. A method of claim 14 wherein the backflow length is measured by using a probe sensitive to properties of the used fluid.

19. A method of one of claims 14 to 18 wherein the result of backflow estimation is used to refine the infusion or the information used within the procedures described in any of claims 1 to 13.

20. A method of claim 19 wherein the refinement is done automatically and is implemented in software.

21. The method according to one of the preceding claims further including the step of checking against one or more reference values and estimating the suitability of the chosen trajectory for the purposes of a fluid infusion.

22. The method according to one of the preceding claims, wherein the computed backflow distance or shape is used to simulate further pressure fields and/or fluid concentration and/or flow parameters.

23. A method of infusing an agent into tissue using the shape or length of the backflow determined according to one of the preceding claims.

24. A method of infusing an agent into tissue according to the preceding claim, wherein the shape or distance of the backflow is computed using altered data, devices, fluids or parameters to refine the determination or simulation of the backflow.

25. Computer program, which, when loaded or running on a computer, performs the method according to one of the preceding claims.

26. Program storage medium or computer program product comprising the program of the preceding claim.

27. Apparatus for determining the backflow along a delivery device track including an imaging device and a data bank each connected to a computer, wherein the computer can perform the method according to one of claims 1 to 25.

28. System for infusing a substance comprising an apparatus for determining the backflow along a delivery device track according to the previous claim and an infusion system connected to the computer.
